Europäisches Patentamt

European Patent Office

Office européen des brevets

(19)

(11) Publication number: **0 001 325**
**B1**

(12)

# EUROPEAN PATENT SPECIFICATION

(45) Date of publication of patent specification:
29.04.81

(21) Application number: 78300295.9

(22) Date of filing: 17.08.78

(51) Int. Cl.³: **C 07 C 33/02**, C 07 C 29/04

(54) **Process for the manufacture of butenediol.**

(30) Priority: 22.09.77 GB 3953377
23.01.78 GB 260078
02.08.78 GB 3201378

(43) Date of publication of application:
04.04.79 Bulletin 79/7

(45) Publication of the grant of the patent:
29.04.81 Bulletin 81/17

(84) Designated Contracting States:
BE DE FR GB NL

(56) References cited:
**None**

(73) Proprietor: IMPERIAL CHEMICAL INDUSTRIES LIMITED,
Imperial Chemical House Millbank, London SW1P 3JF
(GB)

(72) Inventor: Vasey, Charles Henry, 52, Marske Mill Lane,
Saltburn Cleveland (GB)
Inventor: Waddan, Dhafir Yusuf, 35, Linwood Avenue,
Stokesley Middlesbrough Cleveland (GB)
Inventor: McIndoe, Christopher John, 14, Gleneagles
Road, New Marske Redcar Cleveland (GB)

(74) Representative: Taylor, Michael et al, Imperial Chemical
Industries Limited Legal Department: Patents Thames
House North Millbank, London SW1P 4QG (GB)

Note: Within nine months from the publication of the mention of the grant of the European patent, any person may give notice to
the European Patent Office of opposition to the European patent granted. Notice of opposition shall be filed in a written reasoned
statement. It shall not be deemed to have been filed until the opposition fee has been paid (Art. 99(1) European patent
convention).

## Process for the Manufacture of Butenediol

This invention relates to the manufacture of butenediols and more particularly to the manufacture of 2-butene-1,4-diol.

It is known from German OLS 2 607 039 to react an aliphatic olefine, particularly a mono olefine, containing 2 to 4 carbon atoms in an aqueous medium with copper or iron cations and bromine containing anions, optionally in the presence of molecular oxygen so as to produce vicinal glycols such as ehtylene glycol and 1,2-propylene glycol. We have now found that when the olefine is butadiene the product of the reaction includes 2-butene-1, 4-diol, that as well as copper or iron other cations may be used and that the reaction may be carried out in the presence of anions other than bromine containing anions.

Thus according to our invention a process for the manufacture of 2-butene-1,4-diol comprises reacting butadiene with water and oxygen in the presence of a copper, nickel, cobalt, chromium, manganese, iron or molybdenum catalyst.

The reaction is preferably effected in the presence of carbon dioxide or sulphur dioxide and may be effected in either the liquid or the vapour phase.

The stoichiometric amount of water required in the process is one mole per mole of 25 butadiene, but an excess may be used, for example up to 10 moles/mole butadiene.

The oxygen may be molecular oxygen as such or may be a molecular oxygen-containing gas consisting of a mixture of oxygen with another gas which does not interfere with the reaction, for example nitrogen or argon. Air may be used, as may mixtures of oxygen and nitrogen with a higher or lower oxygen content than that of air.

The carbon dioxide or sulphur dioxide, when used, acts as a promoter and need not be used in equimolar amounts compared with that of the butadiene. Amounts of from 0.01 to 0.5 mols of carbon dioxide or sulphur dioxide per mole of butadiene are convenient, although larger amounts are not deleterious and are not excluded.

The catalyst may be the metal or a metal compound or a mixture of the metal and a compound. The metal compound is particularly a salt, for example an inorganic salt such as a halide, for example chloride, bromide or iodide, or an organic acid salt, especially a carboxylate and particularly a salt of an aliphatic carboxylic acid having up to 20 preferably up to 6 carbon atoms. The metal in the metal compound may be in any of the possible valency states. Examples of suitable copper compounds are cuprous chloride, cuprous bromide, cupric acetate, cupric glycollate, cupric lactate and cuprous trifluormethane sulphonate.

Examples of suitable nickel compounds are nickel chloride, nickel bromide, nickel iodide and nickel acetate. Examples of suitable cobalt compounds are cobalt (II) chloride, cobalt (III) chloride, cobalt (II) bromide, cobalt (II) iodide, cobalt (II) acetate and cobalt (II) propionate.

Examples of suitable chromium compounds are chromium (II) chloride, chromium (III) chloride, chromium (III) bromide, chromium (III) iodide, chromium (II) acetate, chromium (III) acetate and chromium (III) acetoacetonate. Examples of suitable manganese compounds are manganese dichloride, manganese trichloride, manganese dibromide, manganese diiodide, manganese (II) benzoate, manganese (II) lactate, manganese (II) tartrate. Examples of suitable molybdenum compounds are molybdenum di-, tri-, tetra- and penta-chlorides, molybdenum di- and tetra-bromides, molybdenum di- and tetra-iodides and molybdenum hexacarbonyl. Among the iron compounds which may be used in the process are included ferrous chloride, ferrous bromide, ferrous or ferric acetate, ferric glycollate, ferric lactate, ferric acetyl-acetonate and ferric stearate.

It is also advantageous to carry out the reaction in the presence of lithium hydroxide.

When the reaction is effected in the liquid phase it is conveniently carried out in the presence of a solvent. The solvent is preferably water-miscible. Suitable solvents are, for example, lower (eg. $C_1$ to $C_6$) aliphatic alcohols, lower (eg. $C_1$ to $C_6$) aliphatic ketones and lower (eg. $C_1$ to $C_6$) alkyl cyanides, for example ethanol, n-propanol, isopropanol, butanols, especially t-butanol, butenediol, acetone, methyl ethyl ketone, methyl isobutyl ketone and acetonitrile. Compounds may be added which may assist the solubility of the butadiene and/or the catalyst in the water used in the reaction. Such compounds are, for example, organic sulphur compounds, for example crotyl phenyl sulphide and triphenyl thio-phosphite.

The reaction is best carried out at a raised temperature, for example at a temperature within the range 50° to 150°C, preferably 80° to 130°C. The reaction may be conveniently carried out, for example, by adding butadiene, water, solvent and catalyst, and optionally carbon dioxide or sulphur dioxide, to an autoclave, pressurising the autoclave with oxygen and heating the autoclave to the appropriate temperature for the desired time. The time of reaction may be varied according to the degree of conversion desired, and may vary, for example, from 0.5 hour up to 50 hours. The butenediol product may be separated from the reaction mixture, for example by fractional distillation. Unconverted materials may be recycled.

The product consists of a mixture of cis- and trans-2-butene-1, 4-diol with some butene-3, 4-diol. The product may be used as an intermediate in the manufacture of tetrahydrofuran, in the manufacture of polyurethanes, and for conversion to butane-1, 4-diol useful in the manufacture of polyesters.

The invention is illustrated but not limited by the following Examples in which the parts and percentages are by weight except where stated otherwise, and in which the ratio of parts by weight to parts by volume is that of the kilogram to the litre.

Examples 1 to 18

Butadiene (6.5 parts), water (5 parts by volume) and the carbon dioxide, catalyst and solvent specified in the following tables were added to an autoclave which was pressurised with oxygen to 6.5 bar (gauge) and heated at 100° for 20 hours. The reaction mixture was found to contain the amount of 2-butene-1, 4-diol specified.

Table 1

Examples 1—8

| Example No. | Catalyst and amount (parts) | | Solvent and amount (parts by vol.) | | Carbon Dioxide (parts) | Yield of 2-butene-1,4-diol (parts) | % Conversion of butadiene to 2-butene-1,4-diol |
|---|---|---|---|---|---|---|---|
| 1 | cuprous bromide<br>copper bronze | 0.25<br>0.25 | acetone<br>Crotyl phenylsulphide | 25<br>5 | 2 | 1.4 | 14 |
| 2 | cuprous bromide<br>copper bronze | 0.25<br>0.25 | acetone | 5 | 2 | 0.15 | 1.4 |
| 3 | cupric acetate<br>copper bronze | 0.25<br>0.25 | acetone<br>Crotyl phenylsulphide | 5<br>0.5 | 1 | 0.58 | 5.8 |
| 4 | cupric acetate<br>copper bronze | 0.25<br>0.25 | acetone<br>Crotyl phenylsulphide | 25<br>2 | 1 | 1.1 | 10 |
| 5 | cupric acetate<br>cupric bronze | 0.25<br>0.25 | acetone<br>Triphenyl thiophosphite | 8<br>0.5*) | 1 | 0.52 | 5.2 |
| 6 | cupric acetate<br>cuprous trifluoromethane sulphonate | 0.25<br>0.1 | acetone | 25 | 1 | 0.65 | 6.4 |
| 7 | cupric acetate<br>cuprous trifluoromethane sulphonate | 0.25<br>0.1 | Isopropanol | 20 | 1 | 0.15 | 1.5 |
| 8 | cupric acetate<br>cuprous trifluoromethane sulphonate | 0.25<br>0.1 | Isopropanol | 20 | Nil | 0.01 | — |

*) parts by weight.

Table 2
Examples 9—18

| Example No. | Catalyst and amount (parts) | | Solvent and amount (parts by vol.) | | Carbon Dioxide (parts) | Yield of 2-butene-1,4-diol (parts) |
|---|---|---|---|---|---|---|
| 9 | Co(II)acetate | 0.25 | t-butanol | 15 | 1 | 0.6 |
| 10 | Mn(II)acetate | 0.25 | t-butanol | 15 | 1 | 0.31 |
| 11 | Nickel acetate | 0.25 | t-butanol | 15 | 1 | 0.58 |
| 12 | Molybdenum hexacarbonyl | 0.25 | t-butanol | 15 | 1 | 0.31 |
| 13 | Cr(III)acetoacetonate | 0.25 | Isopropanol | 15 | 1 | 0.2 |
| 14 | Cr(III)acetoacetonate | 0.25 | Acetone | 15 | 1 | 0.81 |
| 15 | Cr(III)acetoacetonate | 0.25 | t-butanol | 15 | 1 | 0.93 |
| 16 | Cr(III)acetoacetonate | 0.25 | Acetonitrile | 10 | 1 | 0.61 |
| 17 | Cr(III)acetoacetonate lithium hydroxide | 0.25 0.25 | Acetonitrile (time of reaction 8 hr@100° C) | 15 | 1 | 1.1 |
| 18 | Cr(III)acetoacetonate lithium hydroxide | 0.25 0.25 | Acetonitrile (conditions as Ex. 17) | 15 | Nil | 0.37 |

Examples 19 to 21

Butadiene (10 parts by volume), water (5 parts by volume) and the carbon dioxide or sulphur dioxide, catalyst and solvent specified in the following Table were added to an autoclave which was pressurised with oxygen to 3.45 bar (gauge) and heated at 100° for the time shown in the Table. The reaction mixture was found to contain the amount of 2-butene-1, 4-diol specified.

| Example No. | Catalyst and amount (parts) | | Solvent and amount (parts by volume) | | Carbon Dioxide (parts) | Time of Reaction (hrs) | Yield of 2-butene-1,4-diol (parts) | % Conversion of butadiene to 2-butene-1,4-diol |
|---|---|---|---|---|---|---|---|---|
| 19 | iron acetylacetonate lithium hydroxide | 0.25 0.2 | acetone 5% sulphur dioxide solution in acetone | 15 0.5 | — | 10 | 0.6 | 5.8 |
| 20*) | iron acetylacetonate lithium hydroxide | 0.05 0.05 | acetone | 15 | 1 | 20 | 0.5 | 4.9 |
| 21*) | iron acetylacetonate lithium hydroxide | 0.01 0.01 | acetone | 15 | — | 20 | 0.3 | 2.9 |

*) Nitrogen at 3.45 bar (gauge) was also supplied to the autoclave.

## Claims

1. A process for the manufacture of 2-butene-1,4-diol which comprises reacting butadiene with water and oxygen in the presence of a copper, nickel, cobalt, chromium, manganese, iron or molybdenum catalyst.

2. A process according to Claim 1 which is carried out in the presence of carbon dioxide or sulphur dioxide.

3. A process according to Claim 2 in which the amount of carbon dioxide or sulphur dioxide is 0.01 to 0.5 mols carbon dioxide or sulphur dioxide per mole of butadiene.

4. A process according to any one of the preceding claims in which at least 1 mole of water is used per mole of butadiene.

5. A process according to any one of the preceding claims in which the catalyst is an inorganic salt or an organic acid salt.

6. A process according to Claim 5 in which the inorganic salt is a halide and the organic acid salt is a salt of an aliphatic carboxylic acid having up to 20 carbon atoms.

7. A process according to Claim 5 in which the inorganic or organic acid salt is a chloride, bromide, iodide, trifluoromethane sulphonate, glycollate, lactate, acetate, propionate, stearate, benzoate, tartrate or acetylacetonate.

8. A process according to any one of the preceding claims in which lithium hydroxide is present.

9. A process according to any one of the preceding claims which is carried out in the liquid phase in the presence of a solvent.

10. A process according to Claim 9 in which the solvent is a $C_1$ to $C_6$ aliphatic alcohol, $C_1$ to $C_6$ aliphatic ketone or a $C_1$ to $C_6$ alkyl cyanide.

11. A process according to any one of the preceding claims in which an organic sulphur compound is added to the reaction medium to assist the solubility of the butadiene and/or the catalyst in the water used in the reaction.

12. A process according to any one of the preceding claims which is carried out at a temperature in the range 50° to 150° C.

## Patentansprüche

1. Verfahren zur Herstellung von 2-Buten-1,4-diol, das eine Umsetzung von Butadien mit Wasser und Sauerstoff in Gegenwart eines Kupfer-, Nickel-, Kobalt-, Chrom-, Mangan-, Eisen- oder Molybdän-Katalysators umfaßt.

2. Verfahren nach Anspruch 1, daß in Gegenwart von Kohlendioxid oder Schwefeldioxid durchgeführt wird.

3. Verfahren nach Anspruch 2, bei dem die Menge des Kohlendioxids oder des Schwefeldioxids 0,01 bis 0,5 Mol Kohlendioxid oder Schwefeldioxid pro Mol Butadien beträgt.

4. Verfahren nach einem der vorhergehenden Ansprüche, bei dem mindestens 1 Mol Wasser pro Mol Butadien eingesetzt wird.

5. Verfahren nach einem der vorhergehenden Ansprüche, bei dem der Katalysator ein anorganisches Salz oder ein Salz einer organischen Säure ist.

6. Verfahren nach Anspruch 5, bei dem das anorganische Salz ein Halogenid und das Salz einer organischen Säure ein Salz einer aliphatischen Carbonsäure mit bis zu 20 Kohlenstoffatomen ist.

7. Verfahren nach Anspruch 5, bei dem das anorganische Salz oder das Salz einer organischen Säure ein Chlorid, Bromid, Jodid, Trifluormethansulfonat, Glykolat, Lactat, Acetat, Propionat, Stearat, Benzoat, Tartrat oder Acetylacetonat ist.

8. Verfahren nach einem der vorhergehenden Ansprüche, bei dem Lithiumhydroxid vorhanden ist.

9. Verfahren nach einem der vorhergehenden Ansprüche, das in der flüssigen Phase in Gegenwart eines Lösungsmittels durchgeführt wird.

10. Verfahren nach Anspruch 9, bei dem das Lösungsmittel ein $C_1$- bis $C_6$-aliphatischer Alkohol, ein $C_1$- bis $C_6$-aliphatisches Keton oder ein $C_1$- bis $C_6$-Alkylcyanid ist.

11. Verfahren nach einem der vorhergehenden Ansprüche, bei dem zu dem Reaktionsmedium eine organische Schwefelverbindung hinzugegeben wird, um die Löslichkeit des Butadiens und/oder des Katalysators in dem in der Reaktion eingesetzten Wasser zu unterstützen.

12. Verfahren nach einem der vorhergehenden Ansprüche, das bei einer Temperatur im Bereich von 50° bis 150° C durchgeführt wird.

## Revendications

1. Procédé de production du 2-butène-1,4-diol, qui comprend la réaction du butadiène avec de l'eau et de l'oxygène en présence d'un catalyseur au cuivre, au nickel, au cobalt, au chrome, au manganèse, au fer ou au molybdène.

2. Procédé suivant la revendication 1, qui est exécuté en présence de dioxyde de carbone ou de dioxyde de soufre.

3. Procédé suivant la revendication 2, dans lequel la quantité de dioxyde de carbone ou de dioxyde de soufre est de 0,01 à 0,5 mole de dioxyde de carbone ou de dioxyde de soufre par mole de butadiène.

4. Procédé suivant l'une quelconque des revendications précédentes, dans lequel on utilise au moins 1 mole d'eau par mole de butadiène.

5. Procédé suivant l'une quelconque des revendications prédécentes, dans lequel le catalyseur est un sel inorganique ou un sel d'acide organique.

6. Procédé suivant la revendication 5, dans lequel le sel inorganique est un halogénure et le sel d'acide organique est un sel d'un acide carboxylique aliphatique comptant jusqu'à 20 atomes de carbone.

7. Procédé suivant la revendication 5, dans lequel le sel inorganique ou sel d'acide organique est un chlorure, bromure, iodure, trifluorométhanesulfonate, glycolate, lactate, acétate, propionate, stéarate, benzoate, tartrate ou acétylacétonate.

8. Procédé suivant l'une quelconque des revendications précédentes, qui est exécuté en présente d'hydroxyde de lithium.

9. Procédé suivant l'une quelconque des revendications précédentes, qui est exécuté en phase liquide en présence d'un solvant.

10. Procédé suivant la revendication 9, dans lequel le solvant est un alcool aliphatique en $C_1$ à $C_6$, une cétone aliphatique en $C_1$ à $C_6$ ou un cyanure d'alkyle en $C_1$ à $C_6$.

11. Procédé suivant l'une quelconque des revendications précédentes, dans lequel on ajoute un composé organique du soufre au milieu de réaction pour favoriser la solubilité du butadiène et/ou du catalyseur dans l'eau utilisée pour la réaction.

12. Procédé suivant l'une quelconque des revendications précédentes, qui est exécuté à une température de l'intervalle de 50 à 150° C.